Europäisches Patentamt

European Patent Office

Office européen des brevets

(11) Veröffentlichungsnummer: **0 233 600**
**A2**

# EUROPÄISCHE PATENTANMELDUNG

(21) Anmeldenummer: **87101991.5**

(22) Anmeldetag: **12.02.87**

(30) Priorität: **15.02.86 DE 3604868**

(43) Veröffentlichungstag der Anmeldung:
**26.08.87 Patentblatt 87/35**

(84) Benannte Vertragsstaaten:
**AT BE CH DE ES FR GB IT LI LU NL SE**

(51) Int. Cl.⁴: **C07K 7/40 , A61K 37/26**

(71) Anmelder: **BEHRINGWERKE
Aktiengesellschaft
Postfach 1140
D-3550 Marburg 1(DE)**

(72) Erfinder: **Löbermann, Hartmut Dr.
Giessener Strasse 50
D-3556 Weimar(DE)**
Erfinder: **Pâques, Eric Paul Dr.
Schmiedeacker 18
D-3550 Marburg(DE)**
Erfinder: **Heimburger, Norbert Prof. Dr.
Sonnenhang 10
D-3550 Marburg(DE)**

(74) Vertreter: **Meyer-Dulheuer, Karl-Hermann, Dr.
et al
HOECHST Aktiengesellschaft Zentrale
Patentabteilung Postfach 80 03 20
D-6230 Frankfurt/Main 80(DE)**

(54) Insulinderivate, Verfahren zu ihrer Herstellung und ihre Verwendung.

(57) Es werden Insulinderivate der allgemeinen Formeln I und II

$$X-(Y_1-S-S-Y_2-Z-\text{"Insulin"})_m \quad I$$

$$A-S-Y_3-Z-\text{"Insulin"} \quad II$$

worin

X eine physiologisch verträgliche Kohlenstoffverbindung ist,

$Y_1$, $Y_2$ und $Y_3$ unabhängig voneinander eine chemische Bindung, oder eine organisch chemische Verbrückung sind,

S ein Schwefelatom ist,

"Insulin" den biologisch aktiven peptidartigen Anteil eines natürlichen oder semisynthetischen, synthetischen oder gentechnisch hergestellten Insulins oder eines seiner biologisch aktiven Analoga ohne die nicht essentielle Aminofunktion bedeutet,

Z eine für die biologische Aktivität des Insulins nicht essentielle Aminofunktion ist,

A ein Wasserstoffatom oder eine thiolhaltige, physiologisch verträgliche Kohlenwasserstoffverbindung ist und

m eine ganze Zahl von 1 bis 20 ist,

diese Derivate enthaltende Mittel, Verfahren zur Herstellung dieser Derivate sowie ihre Verwendung zur Behandlung von Stoffwechselstörungen beschrieben.

## Insulinderivate, Verfahren zu ihrer Herstellung und ihre Verwendung

Die Erfindung betrifft Insulinderivate, Verfahren zu ihrer Herstellung sowie ihre Anwendung.

Insulin ist ein Polypeptidhormon mit einem Molekulargewicht von etwa 6.000 Dalton. Seine beiden Aminosäureketten A und B sind über Disulfidbrücken miteinander verbunden. Insulin wird als Proinsulinform in den β-Zellen des Pankreas synthetisiert und durch proteolytische Modifizierung unter Abspaltung eines als C-Peptid bezeichneten Teils als aktive Form ausgeschieden. Es besitzt eine wichtige Funktion im Glucosestoffwechsel von Säugern. Neben seiner blutzuckersenkenden Eigenschaft hat es auch Einfluß auf den Protein-und Fettsäurestoffwechsel.

Es ist bekannt, daß Insulin zur Therapie des Diabetes mellitus eingesetzt werden kann. Durch Zusatz von Protamin oder Spermidin oder ähnlichen Verbindungen oder durch Zusatz von Zinksalzen erhält man - schwerlösliche Insulinderivate, die zum Beispiel bei subkutaner Applikation eine langanhaltende Blutzuckersenkung bewirken. Bei diesen langwirkenden Depot-Insulinderivaten können jedoch Nebenwirkungen auftreten. Außerdem werden diese schwerlöschen Insulinverbindungen bei s.c. Applikation häufig ungleich resorbiert.

Im Gegensatz dazu ist "Altinsulin" rasch wirksam. Die Wirkung klingt aber nach wenigen Stunden ab.

Aufgabe der Erfindung ist daher die Entwicklung eines intramuskulär oder subkutan resorbierbaren Insulinderivates, das neben einer guten Löslichkeit die Nachteile des Altinsulins oder der langwirkenden Depot-Insuline nicht oder nur vermindert aufweist.

Es wurde überraschenderweise gefunden, daß durch eine Bindung von Insulin an eine physiologisch verträgliche Kohlenstoffverbindung ein wasserlösliches Insulinderivat hergestellt werden kann, das eine vergleichbar rasche Wirkung wie "Altinsulin" entfaltet, jedoch im Vergleich dazu eine deutlich längere Wirkdauer ähnlich wie ein Depotinsulin besitzt.

Es wurde weiterhin überraschenderweise gefunden, daß durch Kupplung von Insulin an einen Proteinase-Inhibitor ein Insulinderivat hergestellt werden kann, das gegen proteolytische Inaktivierung geschützt ist.

Es wurde weiter überraschenderweise gefunden, daß durch eine Bindung von Insulin an eine physiologisch verträgliche Kohlenstoffverbindung über eine Disulfidbrücke ein Insulinderivat erhalten werden kann, das eine definierte Stöchiometrie von Insulin zu Kohlenstoffverbindung besitzt.

Gegenstand der Erfindung ist ein Insulinderivat der allgemeinen Formel I

$$X\text{-}(Y_1\text{-}S\text{-}S\text{-}Y_2\text{-}Z\text{-}"Insulin")_m \qquad I$$

oder II

$$A\text{-}S\text{-}Y_3\text{-}Z\text{-}"Insulin" \qquad II$$

worin

X eine physiologisch verträgliche Kohlenstoffverbindung ist,

$Y_1$, $Y_2$ und $Y_3$ unabhängig voneinander eine chemische Bindung, oder eine organisch chemische Verbrückung sind,

S ein Schwefelatom ist,

"Insulin" den biologisch aktiven peptidartigen Anteil eines natürlichen oder semisynthetischen, synthetischen oder gentechnisch hergestellten Insulins oder eines seiner biologisch aktiven Analoga ohne die nicht essentielle Aminofunktion bedeutet,

Z eine für die biologische Aktivität des Insulins nicht essentielle Aminofunktion ist,

A ein Wasserstoffatom oder eine thiolhaltige, physiologisch verträgliche Kohlenwasserstoffverbindung ist und

m eine ganze Zahl von 1 bis 20 ist.

Als physiologisch verträgliche Kohlenstoffverbindung können Dextran, Hydroxyethylstärke, Gelatine oder verwandte abgebaute bzw. quervernetzte Kollagenverbindungen, Polyaminosäureverbindungen, Polyoxyethylen oder Polyvinylpyrrolidon und deren Derivate, aber vorzugsweise ein lösliches Polymer, insbesondere ein Polypeptid oder Protein verwendet werden. Die Molekulargewichte liegen zwischen 500 und $1,5 \times 10^6$ Dalton, vorzugsweise aber zwischen 2.000 und 500.000 Dalton.

In einem besonders bevorzugten Insulinderivat wird als physiologisch verträgliche Kohlenstoffverbindung ein Enzym-Inhibitor, besonders ein Proteinase-Inhibitor, beispielsweise alpha$_1$-Antitrypsin, verwendet.

$Y_1$, $Y_2$ und $Y_3$ können unabhängig voneinander eine chemische Bindung oder eine organisch chemische Verbückung, vorzugsweise ein aliphatischer, araliphatischer oder aromatischer Kohlenwasserstoffrest sein, in dem Kohlenstoffatome oder Wasserstoffatome durch Heteroatome, besonders N, O, P oder S ersetzt sein können.

In einem bevorzugten Insulinderivat bedeuten $Y_1$ oder $Y_3$ eine Bindung und enthält $Y_2$ eine -$CH_2$-$CH_2$-CO-Gruppe.

Z bedeutet eine für die biologische Aktivität des Insulins nicht essentielle Aminofunktion, vorzugsweise aber die N-terminale Aminosäure der B-Kette des Insulins.

In einem besonders bevorzugten Insulinderivat wird ein Insulin verwendet, das die folgende N-terminale Sequenz in der B-Kette hat: Phe(1)-Val(2)-Asn(3)-Glu(4)-His(5)-Leu(6).

Z-"Insulin" bedeutet ein natürliches oder ein semisynthetisch, synthetisch oder gentechnisch hergestelltes Insulin oder eines seiner biologisch aktiven Analoga. Es können auch "Insuline" verwendet werden, die in der B-Kette N-terminal oder C-terminal verkürzt oder verlängert worden sind.

Vorzugsweise wird aber ein Insulin verwendet, das als N-terminale Aminosäure in der A-Kette Glycin und in der B-Kette Phenylalanin besitzt.

Besonders bevorzugt werden Human-, Schweine-oder Rinderinsulin verwendet.

"Insulin" bedeutet den biologisch aktiven peptidartigen Anteil von Insulin ohne die nicht essentielle Aminofunktion Z.

Falls A eine thiolhaltige, physiologisch verträgliche Kohlenwasserstoffverbindung bedeutet, kann als solche ein Aliphat, Araliphat oder Aromat verwendet werden, in denen Kohlenstoffatome oder Wasserstoffatome durch Heteroatome, besonders N, O, P oder S ersetzt sein können. Diese Kohlenstoffverbindung enthält von 1 bis 50, vorzugsweise 1 bis 20 Kohlenstoffatome und von 0 bis 30, vorzugsweise 0 bis 15 Heteroatome. In einem bevorzugten Insulinderivat werden Thiopyridin, Cystein, Thiopropionsäure, Thiopyridylcarbonsäure, Mercaptobernsteinsäure, Gluthathion, Cysteamin oder Thiamin verwendet. In einem besonders bevorzugten Insulinderivat wird ein substituierter Pyridylrest, beispielsweise 2-Mercaptopyridin verwendet. Gegenstand der Erfindung ist weiterhin ein Verfahren zur Herstellung eines Insulinderivates der Formel I oder II, dadurch gekennzeichnet, daß eine für die biologische Aktivität des Insulins nicht essentielle Aminofunktion in ein Thiol oder eine die Thiolfunktion in geschützter Form enthaltende Verbindung der Formel III

Schutzgruppe-S-$Y_3$-Z-Insulin III

umgewandelt und gegebenenfalls mit einem Thiol umgesetzt wird, wonach eine Verbindung der Formel I oder II erhalten wird.

Besonders ist Gegenstand der Erfindung ein solches Verfahren, dadurch gekennzeichnet, daß selektiv die N-terminale Aminosäure der A-Kette eines Insulins und das Lysin B-29 der B-Kette eines Insulins mit einer Aminoschutzfunktion versehen und die N-terminale Aminosäure der B-Kette des Insulins mit einer thiolhaltigen oder die Thiolfunktion in einer geschützten Form enthaltenden Verbindung, die mit Aminogruppen reagieren kann, umgesetzt werden, wodurch die Thiolfunktion kovalent an die N-terminale Aminosäure der Insulin-B-Kette gebunden wird, und die Aminoschutzgruppen und gegebenenfalls die Thiolschutzgruppe entfernt werden und gegebenenfalls mit einer Verbindung der Formel X-$Y_1$-SH reagieren gelassen wird.

In einer bevorzugten Verfahrenweise werden zunächst die Aminofunktionen von Glycin-A-1 sowie Lysin-B-29 nach in der Peptidchemie üblichen Methoden selektiv geschützt wie beispielsweise durch Umsetzung mit t-Butyloxycarbonyl-Ak tivesterderivaten unter Einführung der t-Butyloxycarbonylgruppe (Boc), wobei ein als Bis-Boc-Insulin bezeichnetes Derivat entsteht. In einem besonders bevorzugten Verfahren wird t-Butyloxycarbonyl-Hydroxisuccinimidester (Boc-Osu; Chem.Ber. (1975) 108, 2758-2763) verwendet. Dann wird eine Thiolfunktion gegebenenfalls in Form einer Verbindung, die diese in einer geschützten Form enthält, eingeführt. Unter einer geschützten Form der Thiolgruppe versteht man eine Verbindung, in der die Thiolfunktion über eine Disulfidbindung mit einer anderen thiolhaltigen Komponente, wie beispielsweise Pyridinthion, verbunden ist. Die thiolhaltige Komponente kann dann unter reduzierenden Bedingungen abgespalten werden.

In einer bevorzugten Verfahrenweise wird das Bis-Boc-Insulinderivat mit einem Thiol oder eine ein Thiol in geschützter Form enthaltenden Reaktivkomponente, beispielsweise ein Thioalkylimidat, Thiolacton oder Pyridyldithioester umgesetzt. Durch Reaktion der N-terminalen Aminogruppe der intakten oder verkürzten Insulin-B-Kette mit einem thiolhaltigen Aktivester, wobei beispielsweise Ester des Hydroxisuccinimids oder o-oder p-Nitrophenols verwendet werden können, wird die Thiolfunktion eingeführt.

In einer besonders bevorzugten Verfahrenweise wird als thiolhaltige Reaktivkomponente N-Succinimidyl-3-(-2-Pyridyldithio)-propionat verwendet.

3

Anschließend werden nach in der Peptidchemie üblichen Methoden die t-Butyloxycarbonyl(Boc)-Schutzgruppen abgespalten, beispielsweise durch Behandlung mit Trifluoressigsäure oder einem HCl/Essigsäure-Gemisch, aber bevorzugt Trifluoressigsäure.

Das auf die oben beschriebene Weise hergestellte thiolhaltige Insulinderivat wird zur Herstellung eines Insu linderivates der allgemeinen Formel I nun mit einer thiolhaltigen oder die Thiolfunktion in geschützter Form enthaltenden physiologisch verträglichen Kohlenstoffverbindung zusammengebracht. Über Disulfidaustausch zwischen Insulin und Kohlenstoffverbindung, die beide die Thiolfunktion oder diese in einer geschützten Form enthalten, wird ein Insulin-S-S-Kohlenstoffverbindung-Komplex hergestellt.

In einer bevorzugten Verfahrensweise wird als physiologisch verträgliche Kohlenstoffverbindung ein thiolhaltiges Protein, zum Beispiel ein Proteinase-Inhibitor oder thiolhaltiger Träger, verwendet. In einem besonders bevorzugten Verfahren wird alpha,-Antitrypsin verwendet. Es ist jedoch auch möglich, Träger zu verwenden, die zunächst keine Thiolfunktion oder diese in einer geschützten Form enthalten. Solche Trägersubstanzen werden vor der Umsetzung mit thiolfunktionalisiertem Insulin mit thiolhaltigen Reaktivkomponenten, beispielsweise Thiolactonen oder N-Succinimidyl-3-2(Pyridyldithio)propionat (SPDP) umgesetzt; besonders bevorzugt wird die Umsetzung mit SPDP.

Zur Herstellung eines Insulinderivates der allgemeinen Formel II wird das auf die oben beschriebene Weise hergestellte thiolhaltige Insulinderivat mit einer thiolhaltigen oder die Thiolfunktion in geschützter Form enthaltenden physiologisch verträglichen Kohlenstoffverbindung umgesetzt. Über Disulfidaustausch wird eine Insulin-S-S-Kohlenstoffverbindung hergestellt.

In einer bevorzugten Verfahrensweise wird als thiolhaltige Kohlenstoffverbindung beispielsweise Pyridinthion, Cystein, Mercaptopropionsäure, Mercaptoessigsäure, Pyridinthioncarbonsäure, Glutathion, Cysteamin, Mercaptobernsteinsäure oder Thiamin verwendet. In einer besonders bevorzugten Verfahrensweise wird Pyridinthion verwendet.

Ein auf die oben beschriebene Weise hergestellter alpha,-Antitrypsin-S-S-Insulin-Komplex oder Pyridyl-S-S-Insulin-Komplex zeichnet sich besonders dadurch aus, daß die biologische Aktivität des Insulins erhalten bleibt, daß er auch bei physiologischem pH-Wert wasserlöslich und sowohl i.m. als auch subkutan resorbierbar ist, daß er im Falle des alpha,-Antitrypsin-S-S-Insulin-Komplexes gegen proteolytische Inaktivierung geschützt ist, daß eine definierte Stöchiometrie zwischen Insulin und physiologisch verträglicher Substanz vorliegt, und daß die pharmakokinetischen Eigenschaften vorteilhafter als die des Altinsulins oder der langwirkenden Depotinsuline sind.

Die pharmakokinetischen Daten eines alpha,-Antitrpysin-Insulin-Komplexes für intravenöse und subkutane Applikation sind in Tabelle 1 dargestellt.

Die auf die oben beschriebene Weise hergestellten Komplexe zwischen Insulin und alpha,-Antitrypsin oder Pyridinthion können zur Behandlung von Stoffwechselerkrankungen, beispielsweise des Diabetes mellitus, eingesetzt werden.

Diese Insulinverbindungen können in Form von Mitteln, die blutisotinisch und bakterizid gemacht worden sind und gegebenenfalls geeignete physiologisch verträgliche Zusätze und Stabilisatoren enthalten, verabreicht werden.

Die Insulinderivate können parenteral, z.B. i.v., i.m., s.c. oder mittels einer Insulinpumpe appliziert werden.

Die folgenden Beispiel erläutern die Erfindung.


Beispiel 1

Albumin-S-S-Insulin-Komplex

a) Bis-Boc-Insulin

1 g Schweineinsulin wurde in 37,5 ml DMF, 9,5 ml $H_2O$ und 2,7 ml 1 N $NaHCO_3$-Lösung gelöst. An-schließend wurden 930 mg t-Butyloxycarbonyl-Hydroxisuccinimidester, der in 2 ml DMF gelöst war, zugesetzt. Die Mischung wurde 4 Stunden bei Raumtemperatur gerührt, mit 50 % Essigsäure auf pH 6,9 eingestellt und das Lösungsmittel abrotiert. Der ölige Rückstand wurde mit 10 ml Methanol versetzt und anschließend in ca. 250 ml Diethylether eingegossen. Der Niederschlag wurde über eine Glasfritte abgetrennt, mit Essigsäureethylester und Diethylether mehrfach gewaschen und über KOH $P_4O_{10}$ getrocknet. An-

schließend wurde der getrocknete Niederschlag in 6 ml 0,5 % NH₄HCO₃ gelöst und über Sephadex®G-50$_{sf}$ in 0,5 % NH₄HCO₃ gelfiltriert. Die Bis-Boc-Insulin enthaltenden Fraktionen wurden vereinigt und anschließend lyophilisiert. Auswaage: 0,85 g.

b) PDP-Bi-Boc-Insulin

Zu 36 mg Bi-Boc-Insulinderivat in 3 ml 0,1 mol/l Natriumphosphat, 100 mmol/l NaCl pH 7,5 fügte man unter Rühren tropfenweise 300 µl N-Succinimidyl-3-2(Pyridyldithio)propionat(SPDP)-Lösung (20 mmol/l in Ethanol) zu. Die Lcsung ließ man 30 min. bei Raumtemperatur stehen. Das 2-Pyridyl-S-S-CH₂-CH₂-CO-NH-Gly₁-Insulin-bi-BOC (PDP-Bi-Boc-Insulin) trennte man durch Gelfiltration an Sephadex G-50 in 5 g/l NH₄HCO₃ pH 8,5 von Verunreinigungen ab und lyophilisierte anschließend.

c) PDP-Insulin

30mg PDP-Bi-Boc-Insulin löste man in 400 µl Trifluoressigsäure, die 40 µl Anisol enthielt. Die Lösung ließ man unter Feuchtigkeitsausschluß 30 min. bei RT und 15 min bei 4°C stehen. Anschließend goß man die Lösung in etwa 100 ml kalten Ether. Der Niederschlag wurde mehrfach mit Ether gewaschen, in 3 ml 5 g/l NH₄HCO₃ pH 8,5 gelöst und durch Gelfiltration an Sephadex® G-50 von Verunreinigungen befreit. Das reine PDP-Insulin wurde anschließend lyophilisiert. Im UV-Spektrum war nach Reduktion mit 0,5 mmol/l Dithiothreitol (DTE) eine zusätzliche Bande bei $\lambda_{max}$ 343 nm zu erkennen, die freiem Pyridinthion entspricht.

d) Albumin-S-S-Insulin

33 mg Mercaptalbumin wurden in 4 ml 50 mmol/l Kaliumphosphat, 200 mmol/l NaCl pH 8,5 gelöst und mit 3,3 mg PDP-Insulin in 1 ml des gleichen Puffers gemischt. Die Reaktionslösung ließ man 1 h bei RT und 4 h bei 4°C stehen. Der gebildete Albumin-S-S-Insulin-Komplex wurde durch Gelfiltration an Biogel ® P 100 von den Reaktionspartnern abgetrennt. Im SDS-Gel war eine Bande, die einem Produkt von etwa 72.000 Dalton entsprach, zu erkennen, die sich nach Reduktion mit DTE in 2 Banden mit etwa 66.000 Dalton und 6.000 Dalton aufspaltet.

Beispiel 2

PDP-Insulin wurde wie in Beispiel 1 beschrieben hergestellt.

27 mg alpha₁-Antitrypsin wurden in 2 ml einer Lösung, die 50 mmol/l Kaliumphosphat und 200 mmol/l NaCl (pH 8,5) und 25 mmol/l Dithiothreitol enthielt, gelöst. Die Reduktionslösung ließ man 1 h bei 37°C und 1 h bei 4°C stehen und trennte anschließend das Reduktionsmittel durch Gelfiltration an Sephadex ® G-50 in gleichem Puffer ab. 27 mg alpha₁-Antitrypsin-SH in 6 ml obigen Puffers mischte man mit 3,2 mg PDP-Insulin in 1 ml Puffer. Die Behandlung und Aufarbeitung der Komplexmischung wurde wie in Beispiel 1 beschrieben vorgenommen. Im SDS-Gel war eine Bande mit einem MG von etwa 60.000 Dalton zu erkennen. Sie zerfiel nach Reduktion mit DTE in 2 Banden mit Molekulargewichten von eta 54.000 und 6.000 Dalton. Der gereinigte alpha₁-Antitrypsin-S-S-Insulin-Komplex zeigte inhibierende Eigenschaften gegenüber Proteasen wie Chymotrypsin, Trypsin und Elastase.

Beispiel 3

Es wurde ein alpha₁-Antitrypsin-S-S-Insulin-Komplex wie in Beispiel 2 hergestellt, wobei Schweineinsulin durch Humaninsulin ersetzt wurde.

Tabelle 1

Blutzuckerwert im Kaninchen nach Applikation von Altinsulin oder alpha₁-Antitrypsin-Insulin-Komplex in % Abhängigkeit von der Zeit in Prozent des Anfangswertes

| | Intravenös IE/ml/kg | | | | Subkutan IE/ml/kg | | | |
| | 0,5 | | 1,0 | | 0,5 | | 1,0 | |
| t in h | AI | KI | AI | KI | AI | KI | AI | KI |
| --- | --- | --- | --- | --- | --- | --- | --- | --- |
| 0 | 100 | | 100 | | 100 | | 100 | |
| 0,5 | 20 | 34 | 18 | 32 | 45 | 85 | 45 | 85 |
| 1 | 25 | 40 | 20 | 35 | 42 | 58 | 43 | 58 |
| 2 | 45 | 35 | 42 | 30 | 45 | 52 | 45 | 47 |
| 3 | 80 | 45 | 70 | 40 | 37 | 45 | 43 | 35 |
| 4 | 110 | 65 | 92 | 53 | 52 | 45 | 53 | 32 |
| 5 | 125 | 90 | 108 | 74 | 65 | 50 | 78 | 35 |
| 6 | - | - | - | - | 105 | 55 | 80 | 40 |

AI = Altinsulin

KI = Alpha₁-Antitrypsin-S-S-Insulin-Komplex

IE = Internationale Insulineinheit

Jeder Blutzuckerwert stellt den Mittelwert aus der Bestimmung in 4 Kaninchen dar. Es wurde angenommen, daß eine Substanz, die 1 mg Insulin enthält, die blutzuckersenkende Wirkung von 25 IE Insulin besitzt.

Beispiel 4

Es wurde ein alpha₁-Antitrypsin-S-S-Insulin-Komplex wie in Beispiel 2 hergestellt, wobei anstelle von Schweineinsulin Rinderinsulin verwendet wurde.

Beispiel 5

Pankreatischer Trypsininhibitor (PTI)-S-S-Insulin-Komplex

Zunächst wurde PTI nach J.Biol.Chem. 235 (2), 396-404 (1960) mit N-Acetyl-DL-Homocysteine-Thiolacton umgesetzt. Man erhielt einen Thiol-funktionalisierten Inhibitor. Die Umsetzung mit PDP-Insulin zum Komplex wurde wie in Beispiel 1 beschrieben ausgeführt. Der PTI-S-S-Insulin-Komplex zeigte inhibitorische Eigenschaften gegenüber Plasmin und Trypsin. Im SDS-Gel war eine Bande mit einem MG von 12.000 Dalton zu erkennen.

Beispiel 6

Dextran-S-S-Insulin-Komplex

Aminofunktionalisiertes Dextran wurde wie in Beispiel 3 beschrieben mit N-Acetyl-DL-Homocysteinthiolacton umgesetzt. Der Thiolgehalt pro mol Dextran betrug etwa 4 mol Thiolgruppen. Die weitere Umsetzung mit PDP-Insulin zum Komplex wurde wie in Beispiel 1 beschrieben durchgeführt.

Beispiel 7

Cystein-S-S-Insulin-Komplex

PDP-Insulin (25, mg), wie in Beispiel 1 beschrieben, hergestellt und gelöst in 5 ml 50 mmol/l Natriumphosphat und 150 mmol/l NaCl, pH 5,0 wurde mit 0,75 g Cysteinhydrochlorid in 100 $\mu$l desselben Puffers umgesetzt. Die Reaktion wurde durch Messung des freigesetzten Pyridinthions bei 343 nm verfolgt. Das Reaktionsprodukt wurde durch Gelfiltration an Sephadex® G-50$_{sf}$ in 5 g/l $NH_4HCO_3$, pH 8,3 von Verunreinigungn abgetrennt und lyophilisiert.

Beispiel 8

Thiamin-S-S-Insulin-Komplex

25 mg PDP-Insulin in 2 ml 50 mmol/l Natriumphosphat, pH 8,0 wurden mit 0,7 mg Dithiothreitol behandelt. Anschließend wurde mit 3 mg Thiamintetrahydrofurfuryldisulfid in 2 ml obigen Puffers umgesetzt. Das Reaktionsprodukt wurde durch Gelfiltration an Sephadex® G-50$_{sf}$ in 5 g/l $NH_4HCO_3$, pH 8,3 von Verunreinigungen abgetrennt und lyophilisiert.

Die Insulinverbindungen nach Beispiel 5 bis 8 zeigten im Kaninchenmodell eine blutzuckersenkende Wirkung.

**Ansprüche**

1. Insulinderivat der allgemeinen Formel I

X-(Y$_1$-S-S-Y$_2$-Z-"Insulin")$_m$      I

oder II

A-S-Y$_3$-Z-"Insulin"      II

worin

X eine physiologisch verträgliche Kohlenstoffverbindung ist,
Y$_1$, Y$_2$ und Y$_3$ unabhängig voneinander eine chemische Bindung oder eine organisch chemische Verbrükkung sind,
S ein Schwefelatom ist,
"Insulin" den biologisch aktiven peptidartigen Anteil eines natürlichen oder semisynthetischen, synthetischen oder gentechnisch hergestellten Insulins oder eines seiner biologisch aktiven Analoga ohne die nicht essentielle Aminofunktion bedeutet,
Z eine für die biologische Aktivität des Insulins nicht essentielle Aminofunktion ist,
A ein Wasserstoffatom oder eine thiolhaltige, physiologisch verträgliche Kohlenwasserstoffverbindung ist und
m eine ganze Zahl von 1 bis 20 ist.

2. Insulinderivat nach Anspruch 1, dadurch gekennzeichnet, daß Y$_1$, Y$_2$ und Y$_3$ unabhängig voneinander eine chemische Bindung oder ein aliphatischer, araliphatischer oder aromatischer Kohlenwasserstoffrest sind, in dem Kohlenstoffatome oder Wasserstoffatome durch Heteroatome, besonders N, O, P oder S ersetzt sein können.

3. Insulinderivat nach Anspruch 1, dadurch gekennzeichnet, daß X ein lösliches, physiologisch verträgliches Polymer oder Polypeptid ist.

4. Insulinderivat nach Anspruch 1, dadurch gekennzeichnet, daß X ein Proteinaseinhibitor, vorzugsweise alpha 1-Antitrypsin ist.

5. Insulinderivat nach Anspruch 1, dadurch gekennzeichnet, daß A ein Wasserstoffatom oder eine aliphatische, araliphatische oder aromatische Kohlenwasserstoffverbindung ist, in der Kohlenstoffatome oder Wasserstoffatome durch Heteroatome, besonders N, O, P oder S ersetzt sein können.

6. Insulinderivat nach Anspruch 1, dadurch gekennzeichnet, daß A zwischen 1 und 50 Kohlenstoffatome, vorzugsweise aber von 1 bis 20 Kohlenstoffatome und von 0 bis 30 vorzugsweise 0 bis 15 Heteroatome enthält.

7. Insulinderivat nach Anspruch 1, dadurch gekennzeichnet, daß A der Rest von Pyridinthion oder eines substituierten Pyridinthions, eines gegebenenfalls amino-, hydroxi-oder carboxy-substituierten Mercaptoalkans, Cysteins oder eines cysteinhaltigen Peptids oder Thiamins ist.

8. Verfahren zur Herstellung eines Insulinderivats nach Anspruch 1, dadurch gekennzeichnet, daß eine für die biologische Aktivität des Insulins nicht essentielle Aminofunktion in ein Thiol oder eine die Thiolfunktion in geschützter Form enthaltende Verbindung der Formel III

Schutzgruppe-S-$Y_3$-Z-Insulin     III

umgewandelt und gegebenenfalls mit einem Thiol umgesetzt wird.

9. Verfahren nach Anspruch 8 , dadurch gekennzeichnet, daß selektiv die N-terminale Aminosäure der A-Kette eines Insulins und das Lysin B-29 der B-Kette desselben Insulins mit einer Aminoschutzfunktion versehen und die N-terminale Aminosäure der B-Kette des Insulins mit einer thiolhaltigen oder die Thiolfunktion in einer geschützten Form enthaltenden Verbindung, die mit Aminogruppen reagieren kann, umgesetzt werden, wodurch die Thiolfunktion kovalent an die N-terminale Aminosäure der Insulin-B-Kette gebunden wird, und die Aminoschutzgruppen und gegebenenfalls die Thiolschutzgruppen entfernt werden und gegebenenfalls mit einer Verbindung der Formel X-$Y_1$-SH reagieren gelassen wird.

10. Verwendung einer Verbindung nach Anspruch 1 zur Behandlung von Stoffwechselstörungen, insbesondere zur Behandlung von Diabetes mellitus.

Patentansprüche für folgenden Vertragsstaat: ES

1. Verfahren zur Herstellung eines Insulinderivats der allgemeinen Formel I

X-($Y_1$ S-S-$Y_2$-Z-"Insulin")$_m$     I

oder II

A-S-$Y_3$-Z-"Insulin"     II

worin

X eine physiologisch verträgliche Kohlenstoffverbindung ist,
$Y_1$, $Y_2$ und $Y_3$ unabhängig voneinander eine chemische Bindung oder eine organisch chemische Verbrükkung sind,
S ein Schwefelatom ist,
"Insulin" den biologisch aktiven peptidartigen Anteil eines natürlichen oder semisynthetischen, synthetischen oder gentechnisch hergestellten Insulins oder eines seiner biologisch aktiven Analoga ohne die nicht essentielle Aminofunktion bedeutet,
Z eine für die biologische Aktivität des Insulins nicht essentielle Aminofunktion ist,
A ein Wasserstoffatom oder eine thiolhaltige, physiologisch verträgliche Kohlenwasserstoffverbindung ist und
m eine ganze Zahl von 1 bis 20 ist,

dadurch gekennzeichnet, daß eine für die biologische Aktivität des Insulins nicht essentielle Aminofunktion in ein Thiol oder eine die Thiolfunktion in geschützter Form enthaltende Verbindung der Formel III

Schutzgruppe-S-Y$_3$-Z-Insulin     III

umgewandelt und gegebenenfalls mit einem Thiol umgesetzt wird.

2. Verfahren nach Anspruch 1, dadurch gekennzeichnet, daß selektiv die N-terminale Aminosäure der - A-Kette eines Insulins und das Lysin B-29 der B-Kette desselben Insulins mit einer Aminoschutzfunktion versehen und die N-terminale Aminosäure der B-Kette des Insulins mit einer thiolhaltigen oder die Thiolfunktion in einer geschützten Form enthaltenden Verbindung, die mit Aminogruppen reagieren kann, umgesetzt werden, wodurch die Thiolfunktion kovalent an die N-terminale Aminosäure der Insulin-B-Kette gebunden wird, und die Aminoschutzgruppen und gegebenenfalls die Thiolschutzgruppen entfernt werden und gegebenenfalls mit einer Verbindung der Formel X-Y$_1$-SH reagieren gelassen wird.